# EUROPEAN PATENT SPECIFICATION

(11) **EP 1 044 672 B1**
(45) Date of publication and mention of the grant of the patent: **07.03.2007**
(21) Application number: 00108281.7
(22) Date of filing: 14.04.2000
(51) Int. Cl.: A61K 8/06, A61K 8/20, A61K 8/36, A61K 8/44, A61Q 1/02, A61Q 5/00, A61Q 19/00

(54) **Water-in-oil emulsified cosmetic**
Kosmetische Wasser-in-Öl-Emulsion
Emulsion cosmétique eau dans huile

(30) Priority: 16.04.1999 JP 10904899
(43) Date of publication of application: 18.10.2000
(73) Proprietor: SHISEIDO COMPANY LIMITED, Chuo-ku, Tokyo 104-8010 (JP)
(72) Inventor: Omura, Takayuki, c/o Shiseido Research Center 1, Yokohama-shi, Kanagawa 223-8553 (JP)
(74) Representative: TER MEER - STEINMEISTER & PARTNER GbR

(56) References cited:
- EP-A- 0 160 430
- EP-A- 0 500 941
- US-A- 4 606 913

## Description

### Background of the Invention

### 1. Field of the invention

This invention relates in general to a water-in-oil emulsified cosmetic, and more particularly to a water-in-oil emulsified cosmetic which gives moisture to the hair or the skin, is moisturing yet not sticky, and has superior stability over time.

### 2. The Prior Art

For the emulsifier of a conventional water-in-oil (W/O) emulsified cosmetic, polyhydric alcohol fatty ester-based surfactants such as glycerine fatty acid ester and sorbitan fatty acid ester as well as polyoxyalkylene-modified organopolysiloxane-based surfactants are generally used.

However, in a W/O emulsion, separation of the oil phase, which is the continuous phase, tends to occur at lower temperatures due to aggregation of water drops. On the other hand, at higher temperatures water drops tend to merge to increase the particle size and therefore sink, leaving only the oil in the top layer, resulting in separation of the oil phase.

Considering this problem, for the purpose of improving the temperature stability, a method in which a large amount of wax is blended in to increase the viscosity has been used. However, even with this method, the stability at higher temperatures is not sufficient.

Also, since the outer phase of a W/O emulsion is oil, there are advantages such as skin protection and making the skin supple. On the other hand, however, there are problems in terms of usability such as stickiness at the time of use, poor spreadability, and hardness.

US-A-4,606,913 relates to an improved high-internal-phase emulsion of the type comprising an aqueous phase, an oil phase and an emulsifier, wherein an amount sufficient to increase the stability of said emulsion of an electrolyte is contained in said aqueous phase.

Based on the situation described above, the inventors conducted earnest research to solve the aforementioned problem and discovered that a water-in-oil emulsified cosmetic which is prepared by combining a complex obtained by mixing an ampholytic surfactant or semi-polar surfactant and higher fatty acid and inorganic salt and/or amino acid salt allows stable emulsification of a large amount of water in a wide range of oils including non-polar oils such as silicone oil, triglycerides, ester oils, and hydrocarbons, as well as polar oils, and thus provides a water-in-oil emulsified cosmetic which has superior stability over time and superior usability, thus completing the present invention.

The object of the present invention is to provide a water-in-oil emulsified cosmetic which enables blending of a large amount of water, has superior stability over time as well as superior usability.

### Brief Summary of the Invention

That is, the present invention providers a water-in-oil emulsified cosmetic according to claim 1.

### Detailed Description of the Invention

The configuration of the present invention is described below.

The complex used in the present invention is obtained by mixing an ampholytic surfactant or semi-polar surfactant and higher fatty acid. The carboxyl group of the higher fatty acid is bonded to the ampholytic surfactant or semi-polar surfactant to form the complex. The details are described in Japanese unexamined patent publication Tokkai Hei 6-65596 (EP 0 500 941).

In the present invention, since this complex acts as an excellent emulsifier, a superior emulsified composition can be prepared without using other surfactants or emulsifiers.

For the ampholytic surfactant or semi-polar surfactant in the complex, the surfactants are represented by the general formulas (1) - (6) as specified in claim 1.

In the aforementioned general formulas (1)-(6), R₁ is an alkyl group or alkenyl group having 9-21 carbon atoms, preferably an alkyl group or alkenyl group having 11-17 carbon atoms, and more preferably an alkyl group or alkenyl group having 11-13 carbon atoms. If the carbon number is less than 9, then the hydrophilicity is too strong. On the other hand, if it is more than 21, then solubility in water becomes poor. R₂ and R₃ denote alkyl groups or alkenyl group having 10-18 carbon atoms. p denotes an integer 2-4, q denotes an integer 0-3, and s denotes an integer 1 or 2.

For the surfactants of the aforementioned general formulas (1)-(6), commercially available products can be used. Specific examples of commercially available amido betaine ampholytic surfactants of general formula (1) include Lebon 2000 (from Sanyo Chemical Industries, Ltd.) and Anon BDF (from Nippon Oil and Fats Co., Ltd.).

Specific examples of commercially available amido sulfobetaine ampholytic surfactants of general formula (2) include Lonzaine (from Lonza Inc.) and Milataine (from Milanol Co., Ltd.).

Specific examples of commercially available betaine ampholytic surfactants of general formula (3) include Anon BL (from Nippon Oil and Fats Co. , Ltd.) and Dehyton (from Henkel Corporation).

Specific examples of commercially available sulfobetaine ampholytic surfactants of general formula (4) include Ronzaine (from Ronza Co., Ltd.).

Specific examples of commercially available imidazolinium ampholytic surfactants of general formula (5) include Obazolin (from Toho Chemical Industry Co., Ltd.) and Anon GLM (from Nippon Oil and Fats Co., Ltd.).

Specific examples of commercially available tertiary amine oxide semi-polar surfactants of general formula (6) include Unisafe A-LM (from Nippon Oil and Fats Co. , Ltd. ) and Wondamine (from Shin Nihon Rika Co., Ltd.).

Any one or more of these ampholytic surfactants or semi-polar surfactant can be selected for use in the complex of the present invention.

For the higher fatty acid in the present invention, the higher fatty acids are represented by the aforementioned general formula (7).

In the aforementioned general formula (7), R₄ is a linear chain, branched chain, or hydroxide-group-containing saturated or unsaturated hydrocarbon having 7-25 carbon atoms, and more preferable is a linear chain, branched chain, or hydroxide-group-containing saturated or unsaturated hydrocarbon having 11-21 carbon atoms. If the carbon number is less than 7, then the hydrophilicity is too strong and the complex is hard to form. On the other hand, if the carbon number is more than 25, then the melting point is too high and the complex is hard to form.

Specific examples of the higher fatty acid represented by the aforementioned general formula (7) include saturated fatty acids such as lauric acid, myristic acid, palmitic acid, stearic acid, arachic acid, and behenic acid, unsaturated fatty acids such as 2-palmitoic acid, petroseric acid, oleic acid, elaidic acid, ricinolic acid, linolic acid, linoelaidic acid, linolenic acid, and arachidonic acid, branched chain fatty acids such as isostearic acid, and hydroxycarboxylic acids such as 12-hydroxystearic acid.

In the present invention, any one or more of these higher fatty acids are selected and used.

It is preferable, in terms of stability, to adjust the blend ratio between the higher fatty acid and the ampholytic surfactant or semi-polar surfactant in the complex such that the weight ratio of the former against the latter is 0.5 : 9.5 - 9.5 : 0.5 {(higher fatty acid)/(ampholytic surfactant or semi-polar surfactant) = 0.05-19}, more preferably 1 : 9 - 9 : 1 {(higher fatty acid)/(ampholytic surfactant or semi-polar surfactant) = 0.1-9}.

The total amount of the ampholytic surfactant or semi-polar surfactant and the higher fatty acid is, in terms of stability, 0.5-20.0 wt%.

The complex of ampholytic surfactant or semi-polar surfactant and the higher fatty acid used in the present invention can be added to the recipe of the emulsified cosmetic after mixing the two together. However, it is also possible to blend in the ampholytic surfactant or semi-polar surfactant and the higher fatty acid along with other ingredients in the recipe.

Next, the stabilizers in the present invention, i.e. (B) the inorganic salt and/or amino acid salt, are described.

The inorganic salt used in the present invention are sodium chloride, potassium chloride, magnesium chloride, calcium chloride, aluminum chloride, sodium sulfate, potassium sulfate, magnesium sulfate, and aluminum sulfate.

The blend ratio of the inorganic salt used in the present invention is 0.2-5.0 wt%, of the total amount of the emulsified cosmetic. If it is less than 0.1 wt%, then the emulsion cannot be stabilized. There is no increase in the efficacy if the blend ratio increases over 8.0 wt%.

The amino acid salt used in the present invention is selected from sodium aspartate, potassium aspartate, magnesium aspartate, sodium glutamate, potassium glutamate, magnesium glutamate, calcium glutamate, glutamic acid hydrocloride, cysteine hydrochloride, histidine hydrochloride, lysine hydrochloride, ornithine hydrochloride, ornithine acetate, tryptophan hydrochloride, arginine glutamate, ornithine glutamate, lysine glutamate, lysine aspartate, and ornithine aspartate. Of these, sodium glutamate is preferable.

The blend ratio of the amino acid salt used in the present invention is 0.2-5.0 wt%, of the total amount of the emulsified cosmetic. If it is less than 0.1 wt% then the emulsion cannot be stabilized. There is no increase in the efficacy if the blend ratio increases over 8.0 wt%.

For the stabilizer of the present invention, it is sufficient to use one of the aforementioned inorganic salts or amino acid salts, but two or more inorganic salts or amino acid salts can be mixed as well. In such a case, the total blend ratio is 0. 2-5.0 wt%.

Selection of the oil component used in the water-in-oil emulsified cosmetic of the present invention is not limited in particular, and oil components which are normally used in emulsified cosmetics can be used. Examples are widely varied from polar oils to non-polar oils, including fats/oils such as olive oil, coconut oil, safflower oil, castor oil and cottonseed oil, waxes such as lanolin, jojoba oil, carnauba wax, and candelilla wax, hydrocarbon oils such as liquid paraffin, squalane, and petrolatum, fatty acids, ester oils such as cetyl octanoate and isopropyl myristate, silicone oils such as dimethyl polysiloxane, methylphenyl polysiloxane, amino-modified silicone, and fluoride-modified silicone, as well as their gum-like silicone, and perfluoropoly ether.

The blend ratio of the oil component is 5.0-25.0 wt%.

The water content of the water-in-oil emulsified cosmetic of the present invention is 70.0 wt % or more, of the total amount of the water-in-oil emulsified cosmetic. For the present invention, water-in-oil emulsified cosmetics with a high inner water phase are preferable.

In addition to the aforementioned essential ingredients, other ingredients can be blended into the water-in-oil emulsified cosmetic of the present invention as necessary within the quantitative and qualitative range which does not affect the effect of the present invention, and preparation can be conducted with a conventional method. Examples of these ingredients include water soluble polyhydric alcohols such as ethylene glycol, propylene glycol, 1,3-butylene glycol, glycerine, polyglycerine, sorbitol, and polyethylene glycol, humectants such as hyaluronic acid, chondroitin sulfuric acid, and pyrolidone carboxylate, ultraviolet light absorbents, ultraviolet light scattering agents, resins such as acrylic type resins and polyvinylpyrolidone, proteins or hydrolyzed proteins such as soybean protein, gelatin, collagen, silk fibroin, and elastin, preservatives such as ethyl paraben and butyl paraben, various amino acids, activating agents such as biotin and pantothenic acid derivatives, blood circulation promoting agents such as gamma-oryzanol, sodium dextran sulfate, vitamin E derivatives and nicotinic acid derivatives, anti-seborrheic agents such as sulfur and thiantol, diluents such as ethanol and isopropanol, thickeners such as hydroxyethyl cellulose, pharmaceuticals, fragrance preservers, colored pigments, bright pigments, organic powder, hydrophobically treated pigments, and tar pigments.

Examples of the water-in-oil emulsified cosmetic of the present invention include emulsions or cream products such as an emulsion, cream, foundation, eye liner, mascara, and eye shadow.

According to the present invention, a large amount of water can be emulsified in a wide range of oils including silicone oils, triglycerides, ester oils, and hydrocarbons, and a water-in-oil emulsified cosmetic with excellent stability over time and superior usability can be provided.

### Examples

The present invention is described in detail by referring to examples below. The present invention is not limited to these examples. The blend ratios are indicated in weight percent units.

### [Examples 1-4 and Comparative examples 1-4]

Cream, which is an emulsified cosmetic, was prepared using the recipe shown in Table 1 and the stability and usability of the obtained cream was evaluated by means of a stability test and an actual use test by a panel of ten female specialists. The stability test results were obtained by evaluating the appearance after the samples were allowed to stand for one month at 50°C, and the actual use test was conducted by evaluating the preferences during the use, each based on the following evaluation criteria, respectively.

### [Evaluation criteria of the stability]

○ : No separation was observed.
Δ : Almost no separation was observed.
× : Separation of a liquid phase (oil phase or water phase) occurred.

### [Evaluation criteria of the usability]

○ : Seven or more of the ten female specialists in the panel reported the sample was glossy, moist but not sticky, and judged that the usability was good.
Δ : Three or more and less than seven of the ten female specialists in the panel reported the sample was glossy, moist but not sticky, and judged that the usability was good.
× : Less than three of the ten female specialists in the panel reported the sample was glossy, moist but not sticky, and judged that the usability was good.

**Table 1**

| | Example | | | | Comparative example | | |
|---|---|---|---|---|---|---|---|
| | 1 | 2 | 3 | 4 | 1 | 2 | 3 |
| (1) Dimethylpolysiloxane (20mPa·s) | 10.0 | 10.0 | 10.0 | 10.0 | 10.0 | 10.0 | 10.0 |
| (2) Isostearic acid [Trade name: Isostearic acid EX from Kokyu Alcohol Kogyo Co., Ltd. ] | 2.0 | 2.0 | 2.0 | 2.0 | 2.0 | 2.0 | 2.0 |
| (3) Sodium 2-undecyl-N, N, N-(hydroxyethylcarboxymethyl)-2-imidazolin (net 30%) [Trade name: Obazolin 662N from Toho Chemical Industry Co., Ltd.] | 5.0 | 5.0 | 5.0 | 5.0 | 5.0 | 5.0 | 5.0 |
| (4) Ion exchanged water | 77. 0 | 77. 0 | 77. 0 | 77. 0 | 77. 0 | 77. 95 | 63. 0 |
| (5) Ethanol | 5. 0 | 5. 0 | 5. 0 | 5. 0 | 5. 0 | 5. 0 | 5. 0 |
| (6) Sodium glutamate | 1. 0 | - | - | 0. 5 | - | - | 15. 0 |
| (7) Potassium chloride | - | 1.0 | - | - | - | - | - |
| (8) Sodium chloride | - | - | 1.0 | 0.5 | - | 0.05 | - |
| Stability | ○ | ○ | ○ | ○ | × | × | ○ |
| Usability | ○ | ○ | ○ | ○ | ○ | ○ | × |

### <Preparation method>

(1) and (2) were mixed, and the water phase consisting (3), (4), and (5) in which (6) or (7) or (8) was dissolved was gradually added to the oil phase at room temperature by means of a high-speed stirrer (disper) to obtain the target W/O emulsified cosmetic cream.

As indicated in Table 1, the high inner water phase type water-in-oil emulsified cosmetic of the present invention was an excellent cosmetic which was superior in both stability and usability, and gave a fresh sensation during use.

Other examples of the present invention are listed below.

| "Example 5" Skin cream | wt% |
|---|---|
| (1) Liquid paraffin | 6.0 |
| (2) Decamethylcyclopentasiloxane | 10.0 |
| (3) 1,3-butylene glycol | 3.0 |
| (4) Oleic acid | 1.5 |
| (5) Cocoyl amido propyldimethyl glycine (net 30%) [Trade name: Lebon 2000-SF, Sanyo Chemical Industries, Ltd.] | 2.0 |
| (6) Ion exchanged water | 71.3 |
| (7) Sodium glutamate | 3.0 |
| (8) Paraben | 0.2 |
| (9) Antioxidant | Appropriate amount |
| (10) Ethanol | 3.0 |
| (11) Perfume | Appropriate amount |

### <Preparation method>

(1), (2), and (4) were mixed to prepare the oil phase beforehand. The water phase prepared by mixing/stirring/dissolving (3), (5), (6), (7), (9), (10), and (11) was gradually added to the oil phase at room temperature while being stirred by means of a high-speed stirrer (disper) to obtain the target skin cream.

### <Effect>

The same evaluation as for Examples 1-4 was conducted for the obtained skin cream. The usability was excellent (usability evaluation: ○). When applied on the skin, it gave moisture, provided a moisturing sensation and yet was not sticky, and had good stability (stability evaluation: ○).

| "Example 6" Hair treatment cream | wt% |
|---|---|
| (1) Isoparaffin | 3.0 |
| (2) Dimethylpolysiloxane (500 mPa·s) | 5.0 |
| (3) Glycerine | 5.0 |
| (4) Isostearic acid [Trade name: Emery #875 from Emery Co., Ltd.] | 3.0 |
| (5) Lauryl dimethylaminoacetic acid betaine (net 30%) [Trade name: Anon BL from Nippon Oil and Fats Co., Ltd.] | 1.0 |
| (6) Ion exchanged water | 72.9 |
| (7) Ethanol | 8.0 |
| (8) Sodium chloride | 2.0 |
| (9) Paraben | 0.1 |
| (10) Perfume | Appropriate amount |

### <Preparation method>

(1), (2), and (4) were mixed to prepare the oil phase beforehand. The water phase prepared by mixing/stirring/dissolving (3) and (5)-(10) was gradually added to the oil phase at room temperature while being stirred by means of a high-speed stirrer (disper) to obtain the target hair treatment cream.

### <Effect>

The same evaluation as for Examples 1-4 was conducted for the obtained hair treatment cream. The usability was excellent (usability evaluation: ○). When applied on the hair, it gave moisture, provided a moisturing sensation and yet was not sticky, and had good stability (stability evaluation: ○).

| "Example 7" W/0 emulsified foundation | wt% |
|---|---|
| (1) Squalane | 1.0 |
| (2) Dimethylpolysiloxane (6 mPa·s) | 3.0 |
| (3) Propylene glycol | 2.5 |
| (4) Isostearic acid [Trade name: Isostearic acid PK form Kokyu Alcohol Kogyo Co., Ltd.] | 2.5 |
| (5) Sodium 2-undecyl-N,N,N- | |
| (hydroxyethylcarboxymethyl)-2-imidazoline (net 28%) [Trade name: Sofdazoline LHL-saltfree from Kawaken Fine Chemicals Co., Ltd.] | 1.0 |
| (6) Ion exchanged water | 70.9 |
| (7) Ethanol | 1.0 |
| (8) Sodium glutamate | 1.5 |
| (9) Potassium chloride | 1.5 |
| (10) Paraben | 0.1 |
| (11) Titanium dioxide treated with dextrin palmitate | 5. 0 |
| (12) Mica treated with dextrin palmitate | 5.0 |
| (13) Talc treated with dextrin palmitate | 2.5 |
| (14) Iron oxide treated with dextrin palmitate | 2.5 |
| (15) Antioxidant | Appropriate amount |
| (16) Perfume | Appropriate amount |

### <Preparation method>

(1), (2), (4), and (11)-(14) were mixed and dispersed to prepare the oil phase beforehand. The water phase prepared by dissolving (3), (5), (6), (7), (8), (9), (10), (15), and (16) was gradually added to the oil phase at room temperature while being stirred by means of a high-speed stirrer (disper) to obtain the target W/O emulsified foundation.

### <Effect>

The same evaluation as for Examples 1-4 was conducted for the obtained W/O emulsified foundation. The usability was excellent (usability evaluation: ○). When applied on the skin, it gave moisture, provided a moisturing sensation and yet was not sticky, and had good stability (stability evaluation: ○).

## Claims

1. A water-in-oil emulsified cosmetic comprising
(A) 0.5-20 wt% of a complex obtained by mixing:
one or more ampholytic surfactants or semi-polar surfactants selected from the group consisting of surfactants represented by the following general formulas (1)-(6):
(1): Amide betaine ampholytic surfactants 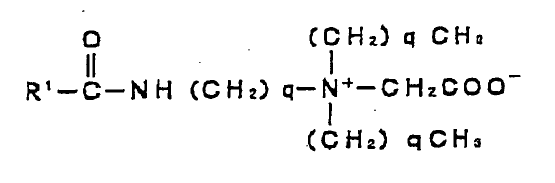
(2): Amide sulfobetaine ampholytic surfactants 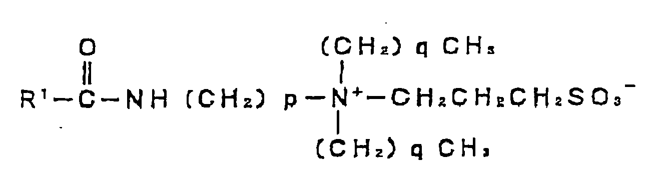
(3): Betaine ampholytic surfactants 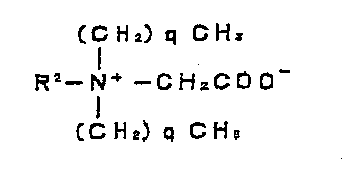
(4): Sulfobetaine ampholytic surfactants 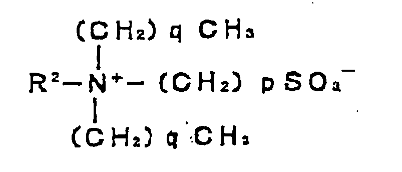
(5): Imidazolinium ampholytic surfactants 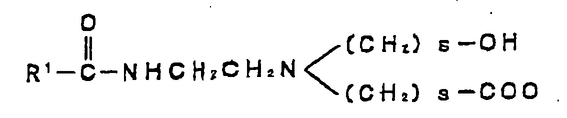
(6): Tertiary amine oxide semi-polar surfactants 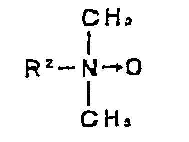
wherein in general formulas (1)-(6), R₁ denotes an alkyl or alkenyl group having 9-21 carbon atoms, R₂ and R₃ denote alkyl or alkenyl groups having 10-18 carbon atoms, p denotes an integer 2-4, q denotes an integer 0-3, and s denotes an integer 1 or 2; and
one or more higher fatty acids represented by the following general formula (7):
R₄COOH (7)
where R₄ denotes a linear chain, branched chain, or hydroxide-group-containing saturated or unsaturated hydrocarbon having 7-25 carbon atoms; and
(B) 0.2-5.0 wt% of one or more inorganic salts selected from the group consisting of sodium chloride, potassium chloride, magnesium chloride, calcium chloride, aluminum chloride, sodium sulfate, potassium sulfate, magnesium sulfate, and aluminum sulfate and/or 0.2-5.0 wt% of one or more amino acid salts selected from the group consisting of sodium aspartate, potassium aspartate, magnesium aspartate, sodium glutamate, potassium glutamate, magnesium glutamate, calcium glutamate, glutamic acid hydrochloride, cysteine hydrochloride, histidine hydrochloride, lysine hydrochloride, ornithine hydrochloride, ornithine acetate, tryptophan hydrochloride, arginine glutamate, ornithine glutamate, lysine glutamate, lysine aspartate, and ornithine aspartate;
(C) 5.0-25.0 wt% of an oil component; and
(D) 70.0 wt% or more water,
of the total amount of the water-in-oil emulsified cosmetic, respectively.

2. The water-in-oil emulsified cosmetic of claim 1 wherein the weight ratio of (higher fatty acid) : (ampholytic surfactant or semi-polar surfactant) in said complex (A) is 0.5 : 9.5 - 9.5 : 0.5.

## Patentansprüche

1. Wasser-in-Öl-Emulsionskosmetikum, umfassend
(A) 0,5 - 20 Gew.-% eines Komplexes, welcher erhältlich ist durch Mischen:
eines oder mehrerer ampholytischer Tenside oder semipolarer Tenside, ausgewählt aus der Gruppe, bestehend aus Tensiden, die durch die folgenden allgemeinen Formeln (1) - (6) dargestellt sind:
(1): ampholytische Amid-Betain-Tenside 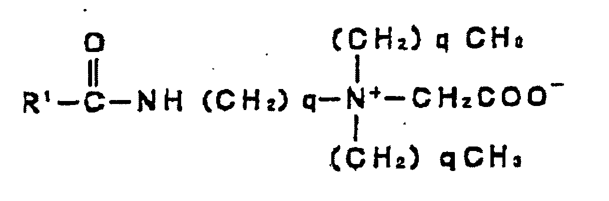
(2): ampholytische Amid-Sulfobetain-Tenside 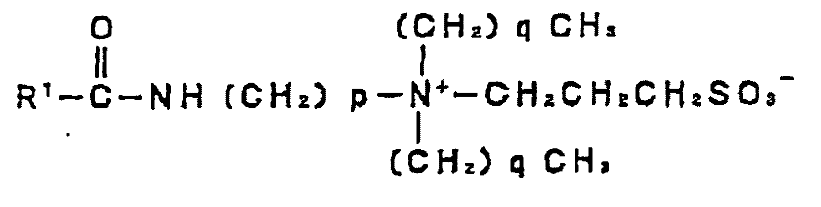
(3): ampholytische Betain-Tenside 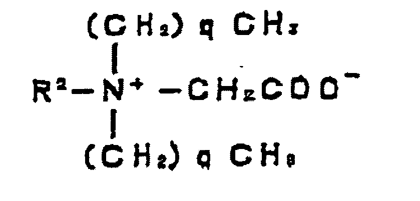
(4): ampholytische Sulfobetain-Tenside 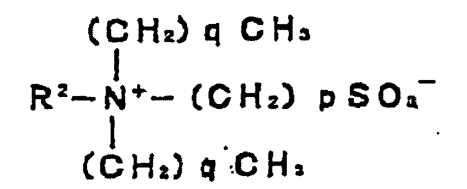
(5): ampholytische Imidazolinium-Tenside 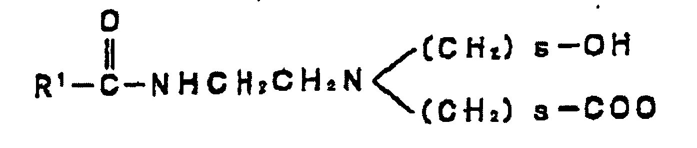
(6): semipolare tertiäre Aminoxid-Tenside 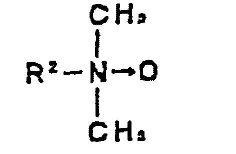
worin in den allgemeinen Formeln (1) - (6) R₁ eine Alkyl- oder Alkenylgruppe mit 9 - 21 Kohlenstoffatomen bedeutet, R₂ und R₃ Alkyl- oder Alkenylgruppen mit 10 - 18 Kohlenstoffatomen bedeuten, p eine ganze Zahl von 2-4 darstellt, q eine ganze Zahl von 0 - 3 bedeutet und s eine ganze Zahl von 1 oder 2 darstellt; und
einer oder mehrerer höherer Fettsäuren, die durch die folgende allgemeine Formel (7) darstellt sind:
R₄COOH (7)
worin R₄ einen geradkettigen, verzweigtkettigen oder Hydroxid-Gruppen enthaltenden gesättigten oder ungesättigten Kohlenwasserstoff mit 7 - 25 Kohlenstoffatomen darstellt; und
(B) 0,2 - 5,0 Gew.-% eines oder mehrerer anorganischer Salze, ausgewählt aus der Gruppe, bestehend aus Natriumchlorid, Kaliumchlorid, Magnesiumchlorid, Calciumchlorid, Aluminiumchlorid, Natriumsulfat, Kaliumsulfat, Magnesiumsulfat und Aluminiumsulfat und/oder 0,2-5,0 Gew.-% eines oder mehrerer Aminosäuresalze, ausgewählt aus der Gruppe, bestehend aus Natriumaspartat, Kaliumaspartat, Magnesiumaspartat, Natriumglutamat, Kaliumglutamat, Magnesiumglutamat, Calciumglutamat, Glutaminsäurehydrochlorid, Cysteinhydrochlorid, Histidinhydrochlorid, Lysinhydrochlorid, Ornithinhydrochlorid, Ornithinacetat, Tryptophanhydrochlorid, Argininglutamat, Ornithinglutamat, Lysinglutamat, Lysinaspartat und Ornithinaspartat;
(C) 5,0 - 25 Gew.-% einer Ölkomponente;
(D) 70,0 Gew.-% oder mehr Wasser;
jeweils des Gesamtgehalts des Wasser-in-Öl-Emulsionskosmetikums.

2. Wasser-in-Öl-Emulsionskosmetikum nach Anspruch 1, worin das Gewichtsverhältnis von (höherer Fettsäure) : (ampholytischem Tensid oder semipolarem Tensid) in dem Komplex (A) 0,5 : 9,5 - 9,5 : 0,5 beträgt.

## Revendications

1. Produit cosmétique émulsionné eau-dans-huile comprenant
(A) 0,5 à 20% en poids d'un complexe obtenu en mélangeant :
un ou plusieurs tensio-actifs ampholytiques ou tensio-actifs semi polaires choisis dans le groupe consistant en les tensio-actifs représentés par les formules générales
(1) à (6) suivantes :
(1) : tensio-actifs ampholytiques de type amide bétaïne 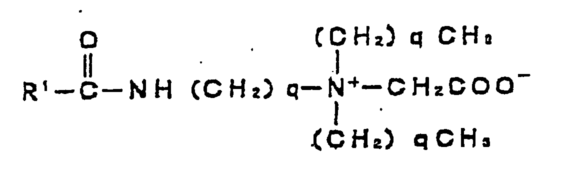
(2) : tensio-actifs ampholytiques de type amide sulfobétaïne 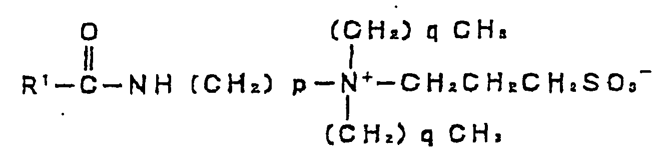
(3) : tensio-actifs ampholytiques de type bétaïne 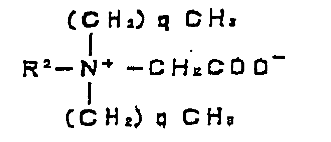
(4) : tensio-actifs ampholytiques de type sulfobétaïne 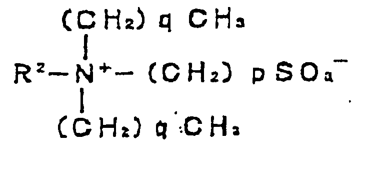
(5) : tensio-actifs ampholytiques de type imidazolinium 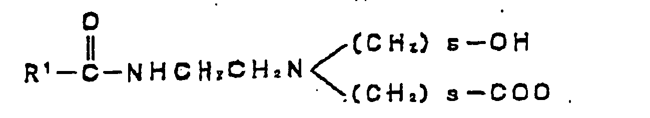
(6) : tensio-actifs semi polaires de type oxyde d'amine tertiaire 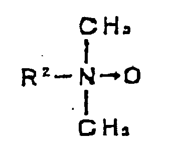
dans lesquels dans les formules générales (1) à (6), R₁ représente un groupe alkyle ou alcényle possédant de 9 à 21 atomes de carbone, R₂ et R₃ représentent des groupes alkyle ou alcényle possédant de 10 à 18 atomes de carbone, p représente un nombre entier de 2 à 4, q représente un nombre entier de 0 à 3, et s représente un nombre entier égal à 1 ou 2 ; et un ou plusieurs acides gras supérieurs représentés par la formule générale suivante
R₄COOH (7)
dans laquelle R₄ représente un radical hydrocarboné saturé ou non saturé, à chaîne linéaire, à chaîne ramifiée, ou contenant un groupe hydroxy, possédant de 7 à 25 atomes de carbone ; et
(B) 0,2 à 5,0% en poids d'un ou plusieurs sels inorganiques choisis dans le groupe consistant en le chlorure de sodium, le chlorure de potassium, le chlorure de magnésium, le chlorure de calcium, le chlorure d'aluminium, le sulfate de sodium, le sulfate de potassium, le sulfate de magnésium, et le sulfate d'aluminium et/ou 0,2 à 5,0% en poids d'un ou plusieurs sels d'acides aminés choisis dans le groupe consistant en l'aspartate de sodium, l'aspartate de potassium, l'aspartate de magnésium, le glutamate de sodium, le glutamate de potassium, le glutamate de magnésium, le glutamate de calcium, le chlorhydrate de l'acide glutamique, le chlorhydrate de cystéine, le chlorhydrate d'histidine, le chlorhydrate de lysine, le chlorhydrate d'ornithine, l'acétate d'ornithine, le chlorhydrate de tryptophane, le glutamate d'arginine, le glutamate d'ornithine, le glutamate de lysine, l'aspartate de lysine, et l'aspartate d'ornithine ;
(C) 5,0 à 25,0% en poids d'un composant huileux ; et
(D) 70,0% en poids ou plus d'eau,
de la quantité totale du produit cosmétique émulsionné eau-dans-huile, respectivement.

2. Produit cosmétique émulsionné eau-dans-huile selon la revendication 1, dans lequel le rapport pondéral (acide gras supérieur) : (tensio-actif ampholytique ou tensio-actif semi polaire) dans ledit complexe (A) est de 0,5 : 9,5 à 9,5 : 0,5.
